# EUROPÄISCHE PATENTSCHRIFT

(11) **EP 3 452 142 B1**
(45) Veröffentlichungstag und Bekanntmachung des Hinweises auf die Patenterteilung: **15.06.2022**
(21) Anmeldenummer: 17718003.1
(22) Anmeldetag: 16.02.2017
(51) Int. Cl.: A61M 1/36, A61M 1/16, A61M 60/562

(54) **ENTLÜFTUNGSSYSTEM MIT EINER ENTLÜFTUNGSEINHEIT UND EINEM ENTLÜFTUNGSVORRICHTUNGSSET SOWIE VERFAHREN ZUM BETREIBEN EINES ENTLÜFTUNGSSYSTEMS**
DEAERATING SYSTEM COMPRISING A DEAERATING UNIT AND A DEAERATING DEVICE SET, AND METHOD FOR OPERATING A DEAERATING SYSTEM
SYSTÈME DE PURGE COMPRENANT UNE UNITÉ DE PURGE ET UN ENSEMBLE DISPOSITIF DE PURGE, AINSI QUE PROCÉDÉ POUR FAIRE UN FONCTIONNER UN SYSTÈME DE PURGE

(30) Priorität: 02.05.2016 DE 102016005338
(43) Veröffentlichungstag der Anmeldung: 13.03.2019
(73) Patentinhaber: Xenios AG, 74076 Heilbronn (DE)
(72) Erfinder: FILIPON, Sven, 74080 Heilbronn (DE); WAGNER, Ozan, 74254 Offenau (DE)
(74) Vertreter: Graf von Stosch, Andreas
(86) Internationale Anmeldenummer: PCT/DE2017/000030
(87) Internationale Veröffentlichungsnummer: WO 2017/190718

(56) Entgegenhaltungen:
- EP-A2- 0 543 172
- WO-A1-97/40867
- WO-A1-2014/162335

## Beschreibung

Die Erfindung betrifft ein Entlüftungssystem, ein Ent- lüftungsvorrichtungsset und ein Verfahren zum Betreiben eines Entlüftungssystems. Das System wird im Folgenden als Entlüftungssystem bezeichnet.

Um ein Schlauchset für eine Kanülierung vorzubereiten, muss sichergestellt werden, dass das Schlauchset mit einer Flüssigkeit gefüllt ist und keine Luft enthält. Der Kreislauf einer Herz-Lungenmaschine wird vor der Befüllung mit Blut mit einer Primingflüssigkeit gefüllt. Je nach Klinik wird vor der Perfusion eine Mischung aus verschiedenen Medikamenten in einen Kreislauf gegeben, der einen Oxygenator, eine oder mehrere Blutpumpen und Verbindungsleitungen aufweist. Eine Primingflüssigkeit kann die folgenden Komponenten aufweisen: Kochsalzlösung 0,9 %, Ringer-Laktat-Lösung, HAES, Mannitol, Heparin, Cortison, Natriumbicarbonat-Lösung, Tranexamsäure.

Für den Priming Vorgang wird ein Priming-Flüssigkeitsbehälter mit der Primingflüssigkeit oberhalb des Primingkreislaufs positioniert, sodass die Primingflüssigkeit in den Primingkreislauf fließt. Anschließend wird der Primingkreislauf an einer geeigneten Stelle geöffnet, sodass die Luft entweichen kann und sich der gesamte Primingkreislauf mit Primingflüssigkeit füllt. Sofern noch Luftblasen im Primingkreislauf sichtbar sind, werden diese durch Schütteln oder Klopfen gelöst, um sicherzustellen, dass sich nach dem Primingvorgang keine Luft mehr im Primingkreislauf befindet.

Dabei ist schwer sicherzustellen, dass tatsächlich alle Blasen aus dem Primingkreislauf entfernt wurden.

Ein gattungsgemäßes Entlüftungssystem ist aus der EP 2 462 967 A1 bekannt. Das dort beschriebene System wurde für eine Herz-Lungen-Maschine entwickelt. Derartige Vorrichtungen werden für chirurgische Operationen verwendet. Sie sind sehr aufwändig im Aufbau und weisen mehrere Drucksensoren, neben dem Primingflüssigkeitsbehälter in einem Bypass mindestens ein Reservoir für Blut aus dem Blutkreislauf und einen arteriellen Filter für kleinere Emboli, Partikel und Knochensplitter auf. Dadurch ist der Systemwiderstand höher als bei Systemen für Therapieverfahren, die im Wesentlichen nur eine Blutpumpe und einen Oxygenator aufweisen.

Um einen Filter besonders langsam mit Blut aus einem Reservoir zu befüllen, wurde vorgeschlagen die Blutpumpe in periodischen Abständen mit einer geringeren Drehzahl zu betreiben. Dadurch wird der Filter zunächst nur zum Teil gefüllt, sodass der obere Bereich der Filtermembran von der Befüllflüssigkeit unbenetzt und luftdurchlässig bleibt. Dadurch können Lufteinschlüsse von der gefilterten auf die ungefilterte Seite übertreten und abgeführt werden. Die eingebrachte Befüllflüssigkeit geht durch den unteren Teil der Filtermembran zur gefilterten Ausgangsseite und füllt das nachgelagerte Kreislaufsystem. Das dabei entstehende Luftpolster im Filter dämpft auf vorteilhafte Weise die einströmende Beftülflüssigkeit in ihrem Strömungsverhalten.

Die EP 0 543 172 A2 offenbart eine Steuervorrichtung für eine medizinische Pumpe und beschreibt deren Verwendung zur Steuerung eines Primingvorgangs. Die WO 2014/162335 A1 beschreibt den Primingbetrieb eines Zirkulationssystems zur extrakorporalen Blutbehandlung, währenddessen Luftblasen im System mittels Luftblasensensor detektiert werden können. Die WO 97/40867 bezieht sich auf eine Herz-Lungen-Maschine mit einem Luftdetektor, der während des Betriebs Luftblasen in der Leitung detektieren kann, um die Luftblasen zu entfernen.

Der Erfindung liegt daher die Aufgabe zugrunde, den Primingvorgang bei Therapieverfahren zu beschleunigen und zu vereinfachen und gleichzeitig die Sicherheit zu erhöhen, dass alle Luftblasen entfernt wurden.

Diese Aufgabe wird mit einem Entlüftungssystem nach Patentanspruch 1, einem Entlüftungsvorrichtungsset nach Patentanspruch 9 und einem Verfahren zum Betreiben eines Entlüftungssystems nach Patentanspruch 10 gelöst. Vorteilhafte Weiterbildungen sind Gegenstand der Unteransprüche.

Das Entlüftungssystem nach Patentanspruch 1 ermöglicht einen automatischen Primingvorgang bei Therapieverfahren. Dabei wird der Luftsensor an einer Stelle des Primingkreislaufs positioniert, an der sich in der Praxis die Luftblasen sammeln. Dabei können auch mehrere Luftsensoren verwendet werden, um automatisch verschiedene Positionen des Kreislaufs auf Luftansammlungen zu überprüfen.

Da die Blutpumpe eine pulsatile Pumpe ist und mit der Steuereinheit in Verbindung steht, können die Luftblasen im Primingkreislauf besonders einfach entfernt werden. Über die Steuereinheit kann die Blutpumpe somit pulsatil betrieben werden, um festhängende Luftblasen in den Schlauchleitungen oder dem Oxygenator zu lösen und aus dem Primingkreislauf zu entfernen. Ein pulsatiles Betreiben führt zu einem Stoß im Moment des Impulses, der die Position einer festhängenden Luftblase verändert. Eine pulsatile Durchströmung bewirkt somit eine kurzzeitige Volumenstromerhöhung und direkt danach eine Absenkung, wobei dieser Vorgang wiederholt durchgeführt wird oder sogar kontinuierlich mit derartigen Schwankungen gearbeitet wird.

Bei einem derartigen System weist üblicherweise der Primingkreislauf kein Reservoir auf.

Außerdem benötigt ein System für Therapieverfahren im Primingkreislauf in Flussrichtung zwischen Oxygenator und Blutpumpe keinen Filter.

Erfindungsgemäß ist zwischen dem Primingflüssigkeitsbehälter und dem Primingkreislauf eine Primingpumpe angeordnet, die mit der Steuereinheit in Verbindung steht. Bei konventionellen Verfahren wird die Primingflüssigkeit ausschließlich über den hydrostatischen Druck zugeführt. Dadurch begrenzt die Behälterhöhe die Flussgeschwindigkeit der Primingflüssigkeit. Die Verwendung einer Primingpumpe ermöglicht es, unabhängig von der Position des Beutels die Geschwindigkeit der Primingflüssigkeit zu variieren, um den Primingvorgang zu beschleunigen. Der Primingflüssigkeitsbehälter kann daher gegebenenfalls auch unterhalb des Primingkreislaufs angeordnet werden. In diesem Fall müsste die Primingpumpe während des Primingvorgangs zumindest mit einem minimalen Durchfluss weiterlaufen, um sicherzustellen, dass das durch die eliminierte Luft im System verlorene Volumen aufgefüllt wird. Außerdem sollte ein Rückfluss aus dem Primingkreislauf in den Primingflüssigkeitsbehälter verhindert werden.

Als Primingpumpe eignet sich eine Rollerpumpe, die durch ihren Aufbau bereits einen automatischen Rückfluss verhindert. Vorteilhaft sind Primingpumpen mit einem Durchfluss von beispielsweise 0,5 bis 10 l/min. Um das System möglichst schnell zu füllen und einen Austritt der Luft zu ermöglichen, wird ein Durchfluss von 1 bis 4 l/min bevorzugt.

Es eignet sich auch eine Pumpe, wie sie in der EP 2 566 533 Al beschrieben ist und die beispielsweise 0 bis 8 l/min bei einem Druck von maximal 550 mmHg fördert. Es können jedoch auch andere in der Medizintechnik verwendbare Kreiselpumpen eingesetzt werden. Bei Kreiselpumpen sollte der Primingflüssigkeitsbehälter oberhalb des Primingkreislaufs angeordnet sein, sofern nicht über einen Nullfluss oder ein Rückschlagventil ein Rückströmen verhindert wird.

Kumulativ und insbesondere alternativ zu einer Primingpumpe steht der Primingflüssigkeitsbehälter erfindungsgemäß mit einem Primingkompressor in Verbindung, um im Primingflüssigkeitsbehälter einen Druck aufzubauen. Der Primingkompressor dient wie die Primingpumpe dazu, den Primingflüssigkeitsvolumenstrom zwischen dem Primingflüssigkeitsbehälter und dem Primingkreislauf zu erhöhen.

Zur Steuerung dieses Volumenstroms kann die Steuereinheit mit dem Primingkompressor in Verbindung stehen, um den Primingkompressor zu regeln.

Kumulativ kann zwischen dem Primingflüssigkeitsbehälter und dem Primingkreislauf eine Durchflusssteuereinheit angeordnet sein, die mit der Primingsteuereinheit in Verbindung steht. Die Durchflusssteuereinheit kann eine Pumpe, ein Ventil oder einen Durchflussbegrenzer aufweisen. Dies ermöglicht es, mit dem Primingkompressor im Primingflüssigkeitsbehälter eines konstanten Primingflüssigkeitsdruck oberhalb des Flüssigkeitsspiegels im Primingflüssigkeitsbehälter zu erzeugen und mittels der Durchflusssteuereinheit den Durchfluss zwischen dem Primingflüssigkeitsbehälter und dem Primingkreislauf zu steuern. Die Durchflusssteuereinheit kann dabei auch pulsierend geöffnet werden, um einen pulsierenden Volumenstrom zu erzeugen, mit dem die Primingflüssigkeit in den Primingkreislauf geführt wird.

Ein vorteilhafter Aufbau des Entlüftungssystems wird dadurch erzielt, dass der Luftsensor im Oxygenator angeordnet ist.

Weiterbildend wird vorgeschlagen, dass der Luftsensor oder ein weiterer Luftsensor in einer der Verbindungsleitungen, vorzugsweise am Eingang der Blutpumpe, angeordnet ist.

Um einen vollautomatischen Ablauf des Primingvorgangs zu gewährleisten und insbesondere auch um Pumpen pulsatil zu betreiben, wird vorgeschlagen, dass die Steuereinheit einen Speicher aufweist, um insbesondere Zeiten für den Betrieb der Blutpumpe zu speichern.

Wenn der Primingflüssigkeitsbehälter über mehr als eine Leitung mit dem Primingkreislauf in Verbindung steht, kann durch eine der Leitungen Primingflüssigkeit aus dem Primingflüssigkeitsbehälter in den Primingkreislauf fließen, während über die andere Leitung Luft oder Primingflüssigkeit mit Luftblasen zurück in den Primingflüssigkeitsbehälter strömt.

Weiterhin wird auch eine Entlüftungseinheit mit einer Steuereinheit beschrieben, die einen Datenprozessor aufweist, der mit Ausgängen an der Entlüftungseinheit für eine Verbindung mit mindestens einem Luftsensor und mit einer Blutpumpe verbunden ist. Eine derartige Entlüftungseinheit dient dazu, bekannte Primingkreisläufe so zu steuern, dass eine vollautomatische Entlüftung erzielt wird.

Vorzugsweise ist der Datenprozessor der Entlüftungseinheit auch mit einem Ausgang an der Entlüftungseinheit für eine Verbindung mit einer Blutpumpe oder einem Kompressor verbunden.

Dabei kann die die Entlüftungseinheit so ausgebildet sein, dass die Primingsteuereinheit mit dem Datenprozessor und die Ausgänge in einem Gehäuse angeordnet sind. Die Entlüftungseinheit bildet somit eine kompakte transportable Vorrichtung, die an einen bekannten Blutkreislauf in der Primingphase angeschlossen werden kann.

Für die praktische Anwendung ist es besonders vorteilhaft, wenn ein Entlüftungsvorrichtungsset eine Entlüftungseinheit und einen Primingflüssigkeitsbehälter als Elemente eines Sets aufweist.

Dieses Entlüftungsvorrichtungsset weist als weiteres Element eine Primingpumpe oder einen Primingkompressor auf.

Bei der Durchführung der Entlüftung ist es besonders vorteilhaft, wenn die Blutpumpe während des Pumpens der Primingflüssigkeit pulsatil betrieben wird.

Um eine sichere Entlüftung zu gewährleisten, wird vorgeschlagen, dass die Steuereinheit vollautomatisch die Entlüftung steuert.

Vorteilhafte Ausführungsvarianten sind in der Zeichnung dargestellt und werden im Folgenden näher erläutert.

### Es zeigt

Figur 1 eine schematische Darstellung eines Entlüftungssystems mit Primingpumpe,
Figur 2 eine schematische Darstellung eines Entlüftungssystems mit Primingkompressor,
Figur 3 eine schematische Darstellung eines nicht erfindungsgemäßen Entlüftungssystems mit hochgelagertem Primingflüssigkeitsbehälter,
Figur 4 schematisch ein Entlüftungssystem mit zwei Blutpumpen und
Figur 5 schematisch den Verfahrensablauf des Verfahrens zum Betreiben des Entlüftungssystems.

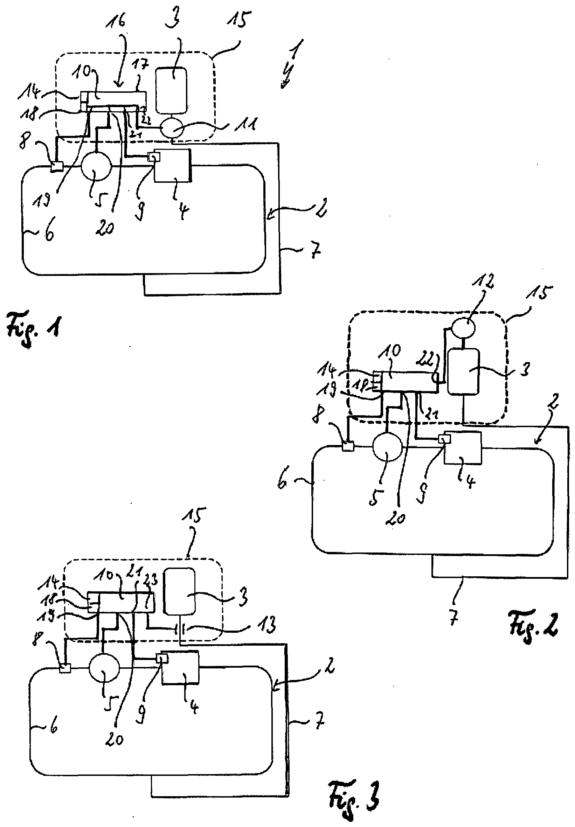

Das in Figur 1 gezeigte Entlüftungssystem 1 weist einen Primingkreislauf 2 und einen Flüssigkeitsbehälter 3 auf. Der Primingkreislauf 2 besteht im Wesentlichen aus einem Oxygenator 4, einer Blutpumpe 5 und Verbindungsleitungen 6. Der Primingflüssigkeitsbehälter 3 steht über eine Leitung 7 mit dem Primingkreislauf 2 in Verbindung.

Der Primingkreislauf 2 hat einen Luftsensor 8 in Flussrichtung des Blutes bzw. der Primingflüssigkeit vor der Blutpumpe 5 und einen weiteren Luftsensor 9 in einem oberen Bereich des Oxygenators 4. Diese Luftsensoren stehen mit einer Primingsteuereinheit 10 in Verbindung, die wiederum mit der Blutpumpe 5 in Verbindung steht.

In dem in Figur 1 gezeigten ersten Ausführungsbeispiel übernimmt die Förderung von Primingflüssigkeit aus dem Primingflüssigkeitsbehälter 3 in den Primingkreislauf 2 eine Primingpumpe 11. Diese Primingpumpe 11 steht mit der Primingsteuereinheit 10 in Verbindung.

In der in Figur 2 gezeigten Ausführungsvariante sind die Elemente des Primingkreislaufs 2 und der Primingflüssigkeitsbehälter 3 im Vergleich zur ersten Ausführungsvariante unverändert. Anstelle der Primingpumpe 11 ist jedoch ein Primingkompressor 12 vorgesehen, der mit dem Primingflüssigkeitsbehälter 3 und der Primingsteuereinheit in Verbindung steht.

Eine nicht erfindungsgemäße Variante ohne Primingpumpe 11 und ohne Primingkompressor 12 ist in Figur 3 dargestellt. Bei diesem Ausführungsbeispiel ist der Primingflüssigkeitsbehälter 3 oberhalb des Primingkreislaufs 2 positioniert, sodass die Primingflüssigkeit nach unten in den Primingkreislauf 2 fließt. Die übrigen Elemente des Entlüftungssystems sind, wie in den Figuren 1 und 2 dargestellt, ausgebildet und angeordnet.

In allen drei Varianten kann zwischen dem Primingflüssigkeitsbehälter 3 und dem Primingkreislauf 2 eine Durchflusssteuereinheit 13 angeordnet sein, die mit der Primingsteuereinheit 10 in Verbindung steht. Diese Durchflusssteuereinheit 13 kann auch nur als Messeinrichtung für Druck oder Volumenstrom ausgebildet sein, um den Durchfluss zwischen Primingflüssigkeitsbehälter 3 und Primingkreislauf 2 zu messen und vorzugsweise an die Primingsteuereinheit 10 weiterzugeben.

Die Primingsteuereinheit 10 weist einen Speicher 14 auf, um beispielsweise Zeiten für den Betrieb der Blutpumpe 5 zu speichern.

Besonders vorteilhaft ist es, wenn die Blutpumpe 5 als pulsatile Blutpumpe ausgebildet ist und von der Steuereinheit 10 angesteuert wird.

Mit strichpunktierter Linie 15 sind die Elemente eines Entlüftungsvorrichtungssets umgrenzt. Dieses Entlüftungsvorrichtungsset weist im Wesentlichen einen Primingflüssigkeitsbehälter 3 und eine Entlüftungseinheit 16 auf. Die Entlüftungseinheit 16 weist ein Gehäuse 17 auf, in dem die Primingsteuereinheit 10 mit dem Speicher 14 und einem Datenprozessor 18 angeordnet ist. Der Datenprozessor 18 steht mit Ausgängen 19, 20, 21 und 22 an der Entlüftungseinheit 16 in Verbindung, um ihn mit den Luftsensoren 8 und 9 und der Blutpumpe 5 zu verbinden. Der Ausgang 22 dient der Verbindung mit der Primingpumpe 11 oder dem Primingkompressor 12. Ein weiterer Ausgang 23 kann für eine Verbindung mit der Durchflusssteuereinheit 13 vorgesehen sein.

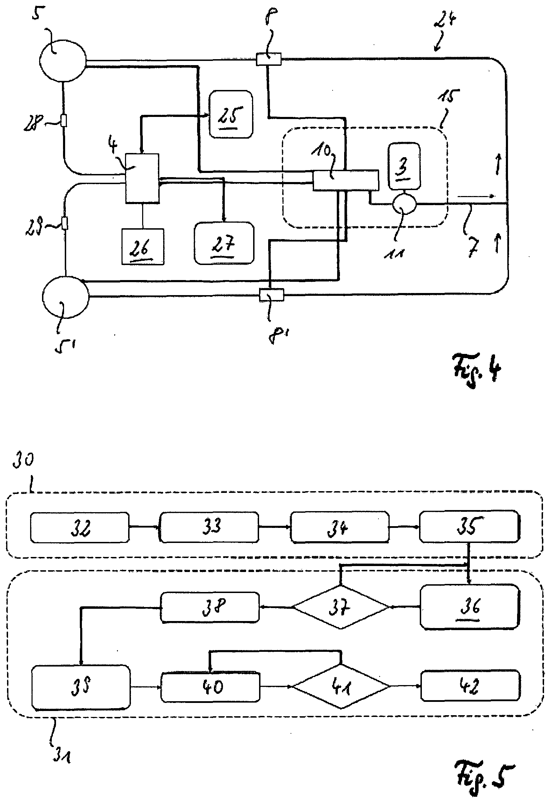

Der Aufbau eines Entlüftungsvorrichtungssets 15 in Verbindung mit zwei Blutpumpen 5 und 5' ist in Figur 4 gezeigt. Das Entlüftungsvorrichtungsset 15 hat eine Verbindungsleitung 7 zu einem Primingkreislauf 24, in dem zwei optische Sensoren 8 und 8' und zwei Blutpumpen 5 und 5' angeordnet sind. Ein Oxygenator 4 im Primingkreislauf 24 steht mit einer Gaszufuhr 25, einer Blutgasanalyse 26 und einem Wärmetauscher 27 in Verbindung. Zwei Drucksensoren 28 und 29 überwachen den Fluss der Primingflüssigkeit durch die Blutpumpen 5 und 5' vor und nach dem Oxygenator 4.

Das Verfahren zum Betreiben eines Entlüftungssystems 1 wird beispielhaft an dem in Figur 1 gezeigten Ausführungsbeispiel im Folgenden näher erläutert.

Die Figur 5 zeigt die einzelnen Verfahrensschritte, wobei die ersten vier mit einer gestrichelten Linie 30 umgrenzten Verfahrensschritte manuell durchgeführt werden und die folgenden mit einer gestrichelten Linie 31 umgrenzten Verfahrensschritte automatisch durchgeführt werden können.

Es beginnt mit dem Verfahrensschritt 32, bei dem der Primingflüssigkeitsbehälter, beispielsweise als Primingbeutel, mit der Entlüftungseinheit 16 über die Primingpumpe 11 gekoppelt wird. Anschließend wird im Verfahrensschritt 33 die Entlüftungseinheit 16 mit den Schlauchsets gekoppelt, das heißt die Entlüftungseinheit wird mit den Luftsensoren 8 und 9 der Blutpumpe 5 und der Primingpumpe 11 verbunden.

Anschließend wird im Verfahrensschritt 34 die Primingsteuereinheit 10 in der Entlüftungseinheit 16 gestartet. Als letzter manueller Schritt 35, der auch automatisch durchgeführt werden kann, wird die Primingpumpe 11 gestartet (in dem in Figur 2 gezeigten Ausführungsbeispiel würde der Kompressor 12 gestartet werden).

Anschließend wird im Verfahrensschritt 36 der Primingkreislauf 2 mit Primingflüssigkeit aus dem Primingflüssigkeitsbehälter 3 gefüllt und dabei der Oxygenator 4 entlüftet. Im Verfahrensschritt 37 wird abgefragt, ob der Sensor 9 im Oxygenator 4 Flüssigkeit erkennt. Sofern er keine Flüssigkeit erkennt, wird über den Verfahrensschritt 36 weiter Primingflüssigkeit in den Primingkreislauf 2 gepumpt.

Erkennt der Sensor 9 im Oxygenator 4 Primingflüssigkeit, wird im Verfahrensschritt 38 die Primingpumpe 11 gestoppt und als Verfahrensschritt 39 wird die Blutpumpe 5 im Kreislauf 2 gestartet und pulsatil betrieben. Im Verfahrensschritt 40 läuft die Blutpumpe 5 im pulsatilen Modus und fördert Restluft in den Oxygenator 4, aus dem die Restluft entweichen kann.

Im Verfahrensschritt 41 prüft der Sensor 8, ob er im Primingkreislauf 2 Luftblasen erkennt. Sofern Luftblasen erkannt werden, geht es mit dem Verfahrensschritt 40 weiter, in dem die Blutpumpe 5 im pulsatilen Betrieb die Restluft zum Oxygenator 4 fördert. Sofern im Verfahrensschritt 41 vom Sensor 8 keine Luftblasen erkannt werden, wird im Verfahrensschritt 42 das Ende der Entlüftung eingeleitet, indem die Blutpumpe 5 gestoppt wird. Danach kann das Entlüftungsvorrichtungsset 15 vom Primingkreislauf 2 abgekoppelt werden.

Nach Verbindung des Entlüftungsvorrichtungssets 15 mit dem Primingkreislauf 2 kann somit vollautomatisch der Primingkreislauf 2 entlüftet werden und, wenn die vollständige Entlüftung an der Entlüftungseinheit 16 angezeigt wird, kann das Entlüftungsvorrichtungsset wieder vom Primingkreislauf entfernt werden.

Als Primingflüssigkeitsbehälter 3 kann ein Primingfllissigkeitsbeutel verwendet werden, der erneuert wird, wenn er leer ist. Der Behälter kann jedoch auch ein starrer Behälter mit einem Zulauf und einem Ablauf und gegebenenfalls mit einer Luftausgleichsleitung sein.

## Patentansprüche

1. Entlüftungssystem (1) mit einem Primingkreislauf (2, 24), der einen Oxygenator (4), eine Blutpumpe (5, 5') und Verbindungsleitungen (6) aufweist, und mit einem Primingflüssigkeitsbehälter (3), der mit dem Primingkreislauf (2, 24) in Verbindung steht, wobei der Primingkreislauf (2, 24) einen Luftsensor (8, 8', 9) und eine Primingsteuereinheit (10) aufweist, wobei der Luftsensor (8, 8', 9) mit der Primingsteuereinheit (10) und die Primingsteuereinheit (10) mit der Blutpumpe (5, 5') in Verbindung steht, wobei die Blutpumpe (5, 5') eine pulsatile Pumpe ist und mit der Primingsteuereinheit (10) in Verbindung steht, um mit der Steuereinheit (10) die Blutpumpe derart pulsatil zu betreiben, dass festhängende Luftblasen in den Schlauchleitungen oder dem Oxygenator gelöst und aus dem Primingkreislauf entfernt werden, ***dadurch gekennzeichnet, dass*** zwischen dem Primingflüssigkeitsbehälter (3) und dem Primingkreislauf (2, 24) eine Primingpumpe (11) angeordnet ist, die mit der Primingsteuereinheit (10) in Verbindung steht, oder der Primingflüssigkeitsbehälter (3) mit einem Primingkompressor (12) in Verbindung steht, um im Primingflüssigkeitsbehälter (3) einen Druck aufzubauen, und die Primingsteuereinheit (10) mit dem Primingkompressor (12) in Verbindung steht, um den Primingkompressor (12) zu regeln.

2. Entlüftungssystem nach Anspruch 1, ***dadurch gekennzeichnet, dass*** der Primingkreislauf (2, 24) kein Reservoir für Blut aufweist.

3. Entlüftungssystem nach einem der vorhergehenden Ansprüche, ***dadurch gekennzeichnet, dass*** der Primingkreislauf in Flussrichtung zwischen Oxygenator (4) und Blutpumpe (5, 5') keinen Filter aufweist.

4. Entlüftungssystem nach einem der vorhergehenden Ansprüche, ***dadurch gekennzeichnet, dass*** zwischen dem Primingflüssigkeitsbehälter (3) und dem Primingkreislauf (2, 24) eine Durchflusssteuereinheit (13) angeordnet ist, die mit der Primingsteuereinheit (10) in Verbindung steht.

5. Entlüftungssystem nach einem der vorhergehenden Ansprüche, ***dadurch gekennzeichnet, dass*** der Luftsensor (9) im Oxygenator (4) angeordnet ist.

6. Entlüftungssystem nach einem der vorhergehenden Ansprüche, ***dadurch gekennzeichnet, dass*** der Luftsensor (8, 8') oder ein weiterer Luftsensor in einer der Verbindungsleitungen (6), vorzugsweise am Eingang der Blutpumpe (5, 5'), angeordnet ist.

7. Entlüftungssystem nach einem der vorhergehenden Ansprüche, ***dadurch gekennzeichnet, dass*** die Primingsteuereinheit (10) einen Speicher (14) aufweist, um insbesondere Zeiten für den Betrieb der Blutpumpe (5, 5') zu speichern.

8. Entlüftungssystem nach einem der vorhergehenden Ansprüche, ***dadurch gekennzeichnet, dass*** der Primingflüssigkeitsbehälter (3) über mehr als eine Leitung (7) mit dem Primingkreislauf (2, 24) in Verbindung steht.

9. Entlüftungsvorrichtungsset (15) für ein Entlüftungssystem (1) nach einem der vorhergehenden Ansprüche 1 bis 8, ***dadurch gekennzeichnet, dass*** es
a) eine Entlüftungseinheit (16) mit der Primingsteuereinheit (10), die einen Datenprozessor (18) aufweist, der mit Ausgängen (19, 20, 21, 22) an der Entlüftungseinheit (16) für eine Verbindung mit mindestens dem Luftsensor (8, 8', 9), für eine Verbindung mit der Blutpumpe (5, 5') sowie für eine Verbindung mit der Primingpumpe (11) oder dem Primingkompressor (12) verbunden ist;
b) den Primingflüssigkeitsbehälter (3); und
c) die Primingpumpe (11) oder den Primingkompressor (12) als Elemente eines Sets aufweist.

10. Verfahren zum Betreiben eines Entlüftungssystems (1) nach einem der vorhergehenden Ansprüche 1 bis 8 während des Pumpens von Primingflüssigkeit, ***dadurch gekennzeichnet, dass*** die Blutpumpe (5, 5') während des Pumpens einer Primingflüssigkeit pulsatil betrieben wird.

11. Verfahren nach Anspruch 10, ***dadurch gekennzeichnet, dass*** die Primingsteuereinheit (10) vollautomatisch die Entlüftung steuert.

## Claims

1. A venting system (1) comprising a priming circulation (2, 24), which comprises an oxygenator (4), a blood pump (5, 5') and connecting tubes (6), and which is connected with a priming fluid container (3), which is connected to the priming circulation (2, 24), wherein the priming circulation (2, 24) has an air sensor (8, 8', 9) and a priming control unit (10), wherein the air sensor (8, 8', 9) is in connection with the priming control unit (10) and the priming control unit (10) is in connection with the blood pump (5, 5'), wherein the blood pump (5, 5') is a pulsatile pump and is in connection with the priming control unit (10), in order to operate, with the priming control unit (10), the blood pump in such a pulsatile manner, that air bubbles that are stuck in the tubing lines or the oxygenator are released and removed from the priming circulation,
***characterized in that*** a priming pump (11), which is in connection with the priming control unit (10), is arranged between the priming fluid container (3) and the priming circulation (2, 24), or the priming fluid container (3) is in connection with a priming compressor (12) for building up pressure in the priming fluid container (3), and the priming control unit (10) is in connection with the priming compressor (12) to regulate the priming compressor (12).

2. The venting system according to claim 1, ***characterized in that*** the priming circulation (2, 24) does not comprise a reservoir for blood.

3. The venting system according to any one of the preceding claims, ***characterized in that*** the priming circulation has no filter in the direction of flow between the oxygenator (4) and the blood pump (5, 5').

4. The venting system according to any one of the preceding claims, ***characterized in that*** a throughflow measuring unit (13) is arranged between the priming fluid container (3) and the priming circulation (2, 24), which is in connection with the priming control unit (10).

5. The venting system according to any one of the preceding claims, ***characterized in that*** the air sensor (9) is arranged in the oxygenator (4).

6. The venting system according to any one of the preceding claims, ***characterized in that*** the the air sensor (8, 8') or a further air sensor is arranged in one of the connecting tubes (6), preferably at the inlet of the blood pump (5, 5').

7. The venting system according to any one of the preceding claims, ***characterized in that*** the priming control unit (10) has a memory (14) in particular for storing times for the operation of the blood pump (5, 5').

8. The venting system according to any one of the preceding claims, ***characterized in that*** the priming fluid container (3) is in connection with the priming circuit (2, 24) by way of more than one tubing line (7).

9. A venting device set (15) for a venting system (1) according to any one of the preceding claims 1 to 8, ***characterized in that*** it comprises
a) a venting unit (16) with the priming control unit (10) having a data processor (18), which is connected to outputs (19, 20, 21, 22) on the venting unit (16) for connection to the air sensor (8, 8', 9), for connection to the blood pump (5, 5') and for connection to the priming pump (11) or the priming compressor (12);
b) the priming fluid container (3); and
c) the priming pump (11) or the priming compressor (12)
as elements of a set.

10. Method for operating a venting system (1) according to any one of the preceding claims 1 to 8 during pumping of priming fluid, ***characterized in that*** the blood pump (5, 5') is operated in a pulsatile manner during the pumping of a priming fluid.

11. The method according to claim 10, ***characterized in that*** the priming control unit (10) controls the venting fully automatically.

## Revendications

1. Système de purge (1) doté d'un circuit d'amorçage (2, 24) présentant un oxygénateur (4), une pompe à sang (5, 5') et des conduites de raccordement (6), et d'un réservoir de liquide d'amorçage (3) raccordé au circuit d'amorçage (2, 24), dans lequel le circuit d'amorçage (2, 24) présente un capteur d'air (8, 8', 9) et une unité de commande d'amorçage (10), dans lequel le capteur d'air (8, 8', 9) est raccordé à l'unité de commande d'amorçage (10) et l'unité de commande d'amorçage (10) est raccordée à la pompe à sang (5, 5'), dans lequel la pompe à sang (5, 5') est une pompe pulsatile et est raccordée à l'unité de commande d'amorçage (10) afin de faire fonctionner la pompe à sang de manière pulsatile avec l'unité de commande (10) de sorte que des bulles d'air coincées dans la tubulure ou l'oxygénateur sont libérées et retirées du circuit d'amorçage, **caractérisé en ce qu'**une pompe d'amorçage (11) raccordée à l'unité de commande d'amorçage (10) est disposée entre le réservoir de liquide d'amorçage (3) et le circuit d'amorçage (2, 24), ou le réservoir de liquide d'amorçage (3) est raccordé à un compresseur d'amorçage (12) afin d'établir une pression dans le réservoir de liquide d'amorçage (3), et l'unité de commande d'amorçage (10) est raccordée au compresseur d'amorçage (12) afin de réguler le compresseur d'amorçage (12).

2. Système de purge selon la revendication 1, **caractérisé en ce que** le circuit d'amorçage (2, 24) est dépourvu de réservoir à sang.

3. Système de purge selon l'une des revendications précédentes, **caractérisé en ce que** le circuit d'amorçage ne présente pas de filtre dans le sens d'écoulement entre l'oxygénateur (4) et la pompe à sang (5, 5').

4. Système de purge selon l'une des revendications précédentes, **caractérisé en ce qu'**une unité de commande de débit (13) raccordée à l'unité de commande d'amorçage (10) est disposée entre le réservoir de liquide d'amorçage (3) et le circuit d'amorçage (2, 24).

5. Système de purge selon l'une des revendications précédentes, **caractérisé en ce que** le capteur d'air (9) est disposé dans l'oxygénateur (4).

6. Système de purge selon l'une des revendications précédentes, **caractérisé en ce que** le capteur d'air (8, 8') ou un autre capteur d'air est disposé dans l'une des conduites de raccordement (6), de préférence à l'entrée de la pompe à sang (5, 5').

7. Système de purge selon l'une des revendications précédentes, **caractérisé en ce que** l'unité de commande d'amorçage (10) présente une mémoire (14) pour notamment mémoriser des temps de fonctionnement de la pompe à sang (5, 5').

8. Système de purge selon l'une des revendications précédentes, **caractérisé en ce que** le réservoir de liquide d'amorçage (3) est raccordé au circuit d'amorçage (2, 24) par plus d'une conduite (7).

9. Ensemble de dispositif de purge (15) pour un système de purge (1) selon l'une quelconque des revendications précédentes 1 à 8, **caractérisé en ce qu'**il présente, comme éléments d'un ensemble,
a) une unité de purge (16) avec l'unité de commande d'amorçage (10), qui présente un processeur de données (18) raccordé à des sorties (19, 20, 21, 22) sur l'unité de purge (16) pour un raccordement à au moins le capteur d'air (8, 8', 9), pour un raccordement à la pompe à sang (5, 5') ainsi que pour un raccordement à la pompe d'amorçage (11) ou au compresseur d'amorçage (12) ;
b) le récipient de liquide d'amorçage (3) ; et
c) la pompe d'amorçage (11) ou le compresseur d'amorçage (12).

10. Procédé de fonctionnement d'un système de purge (1) selon l'une des revendications précédentes 1 à 8 lors du pompage d'un liquide d'amorçage, **caractérisé en ce que** la pompe à sang (5, 5') est actionnée de manière pulsatile lors du pompage d'un liquide d'amorçage.

11. Procédé selon la revendication 10, **caractérisé en ce que** l'unité de commande d'amorçage (10) commande la purge de manière entièrement automatique.
